# EUROPEAN PATENT APPLICATION

(11) **EP 4 729 519 A1**
(43) Date of publication of application: **22.04.2026**
(21) Application number: 24822785.2
(22) Date of filing: 14.06.2024
(51) Int. Cl.: C07D 487/04, A61K 31/18, A61K 31/4188

(54) **CRYSTAL FORM OF ENPP1 INHIBITOR**

(30) Priority: 15.06.2023 CN 202310706966
(71) Applicant: CSPC Zhongqi Pharmaceutical Technology (Shijiazhuang) Co., Ltd., Shijiazhuang, Hebei 050035 (CN); Innovstone Therapeutics Limited, Shanghai 201203 (CN)
(72) Inventor: ZHOU, Fugang, Shijiazhuang, Hebei 050035 (CN); ZHANG, Qichao, Shijiazhuang, Hebei 050035 (CN); SONG, Yunlong, Shanghai 201203 (CN); LU, Kai, Shanghai 201203 (CN); GONG, Zhencai, Shijiazhuang, Hebei 050035 (CN); SHI, Kai, Shijiazhuang, Hebei 050035 (CN); LEI, Xiang, Shijiazhuang, Hebei 050035 (CN); LIU, Chunlei, Shijiazhuang, Hebei 050035 (CN); YANG, Xinxin, Shijiazhuang, Hebei 050035 (CN); KOU, Hongyan, Shanghai 201203 (CN)
(74) Representative: Mewburn Ellis LLP
(86) International application number: PCT/CN2024/099188
(87) International publication number: WO 2024/255837

(57) **Abstract**

Provided in the present application are a compound as represented by formula (I) in a crystalline form, a specific crystal form thereof, a pharmaceutical composition comprising same and the use thereof. The compound as represented by formula (I) in a crystalline form and the specific crystal form thereof have a good crystallinity and chemical purity, are simple and convenient to prepare, and have a high yield and a good druggability potential.

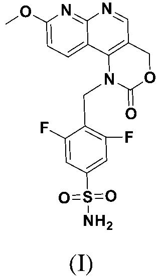

## Description

The present application claims the priority to the prior application filed before the China National Intellectual Property Administration on June 15, 2023 with the patent application No. 202310706966.6 and entitled "CRYSTAL FORM OF ENPP1 INHIBITOR", the content of which is incorporated herein by reference in its entirety.

### TECHNICAL FIELD

The present invention belongs to the field of pharmaceutical chemistry, discloses a crystal form of an ENPP1 inhibitor, and also discloses a use of the crystal form in the preparation of a medicament for treating diseases associated with ENPP1.

### BACKGROUND ART

ENPP1 is a type II transmembrane glycoprotein with nucleotide pyrophosphatase and phosphodiesterase activities, belonging to the ectonucleotide pyrophosphatase/phosphodiesterase (ENPP) family, which consists of seven functionally distinct proteins (1-7). The structure of ENPP1 has two N-terminal SMB domains (SMB1 and SMB2), two linker regions (L1 and L2), a catalytic domain, and a nuclease-like domain. ENPP1 is differentially expressed in immune cells, with low levels in NK cells, DCs, and macrophages, and high levels in neutrophils. ENPP1 is also expressed in a small subset of B cells that may be involved in regulating T cell activity. Its expression is elevated in M2 subtype macrophages, which play a role in tumour promotion. The expression of ENPP1 increases in astrocytoma, breast cancer, and head and neck tumours, and varies greatly among different tumour tissues. The specific high expression of ENPP1 in some tumours may be a driving factor for tumour immune escape and metastasis.

ENPP1 plays an important role in the immune response to multiple stimuli through the STING pathway, achieving high selectivity by selectively activating the STING signaling pathway in tumour cells and other cells in the tumour microenvironment. ENPP1 can also catalyze the hydrolysis of ATP to PPi and AMP, promoting the generation of adenosine, and adenosine has a strong immunosuppressive effect. Inhibiting ENPP1 can reduce adenosine production by inhibiting ATP hydrolysis, thereby lifting tumour immunosuppression.

While STING agonists non-selectively activate STING in cancer cells and host cells, ENPP1 limits the scope of STING activation to tumour tissues and the tumour microenvironment, enhancing anti-tumour immunity. Moreover, ENPP1 is selectively upregulated in metastatic and chromosomally unstable tumour cells. Systemic administration of ENPP1 inhibitors can interfere with the ability of diffused tumour cells to evade immune surveillance, bypassing the technical difficulties of intra-tumour administration of STING agonists.

The combination of ENPP1 inhibitors and radiotherapy has achieved good results in animal models, and there is also a certain synergistic effect when used in combination with a variety of anti-tumour drugs, such as immune checkpoint inhibitors such as PD-1, and PARP inhibitors. Currently, the development of ENPP1 inhibitors is still in the preclinical research stage, but several pharmaceutical companies have published patents on ENPP1 inhibitors, such as WO2021061803A1, WO2021158829A1, WO2020190912A1, WO2019177971A1 and WO2019046778A1.

Although some small molecule ENPP1 inhibitors have been disclosed in existing technologies, there are currently no clinically approved small molecule drugs on the market. Therefore, there is still an urgent need to develop new compounds with better efficacy and pharmacokinetic results that have the potential to be marketed. This invention designs a series of compounds with new structures represented by general formulae and finds that compounds with such structures exhibit excellent effects and functions, which is of positive significance for the development of ENPP1 inhibitors.

### SUMMARY OF THE INVENTION

In a first aspect, the present invention provides a crystal form of a compound of formula (I),

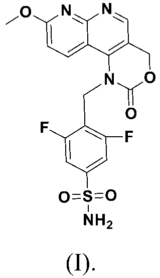

In a preferred aspect of the present invention, the crystal form of the compound of formula (I) is a crystal form A, which has diffraction peaks at the following 2θ angles (±0.2°): 24.9, 25.1, and 25.7 in an X-ray powder diffraction pattern with Cu-Kα radiation.

In some embodiments of the present invention, the crystal form A of the compound of formula (I) above has diffraction peaks at the following 2θ angles (±0.2°): 12.8, 14.0, 16.0, 18.5, 24.9, 25.1, and 25.7 in an X-ray powder diffraction pattern with Cu-Kα radiation.

In some embodiments of the present invention, the crystal form A of the compound of formula (I) above has diffraction peaks at the following 2θ angles (±0.2°): 12.8, 14.0, 16.0, 18.5, 24.9, 25.1, 25.7, 26.9, and 28.5 in an X-ray powder diffraction pattern with Cu-Kα radiation.

In some embodiments of the present invention, the crystal form A of the compound of formula (I) above has diffraction peaks at the following 2θ angles (±0.2°): 12.8, 14.0, 16.0, 18.5, 24.9, 25.1, 25.7, 26.9, 28.5, and 29.8 in an X-ray powder diffraction pattern with Cu-Kα radiation.

In some embodiments of the present invention, the crystal form A of the compound of formula (I) above has diffraction peaks at the following 2θ angles (±0.2°): 12.8, 14.0, 16.0, 17.0, 18.5, 20.5, 24.9, 25.1, 25.7, 26.9, 28.5, and 29.8 in an X-ray powder diffraction pattern with Cu-Kα radiation.

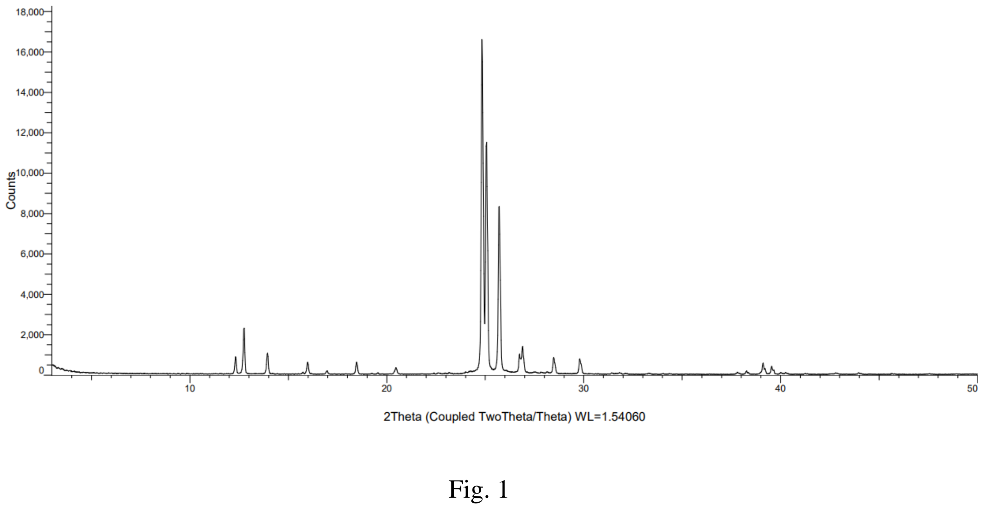

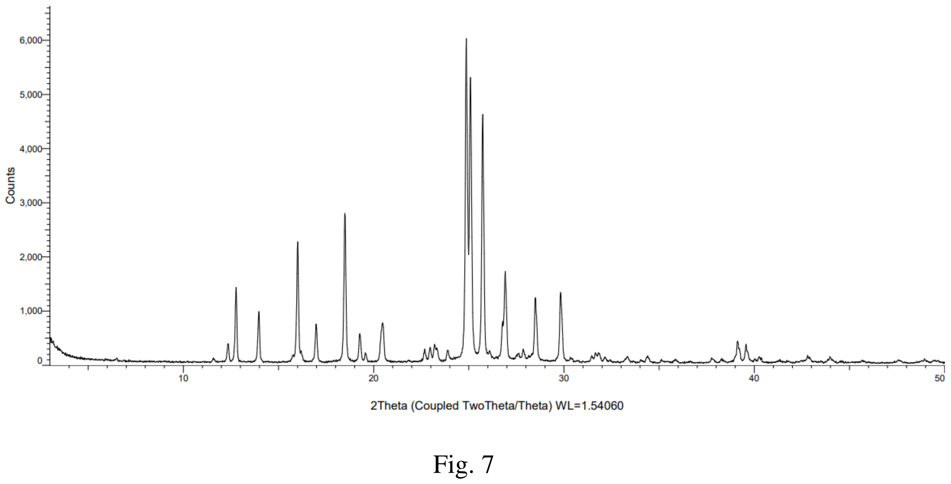

In some embodiments of the present invention, the crystal form A of the compound of formula (I) above has an XRPD pattern with Cu-Kα radiation as shown in Figure 1 or Figure 7.

In some embodiments of the present invention, the crystal form A of the compound of formula (I) above has an XRPD pattern analysis with Cu-Kα radiation as shown in Table 1 or Table 2.

In some embodiments of the present invention, the crystal form A of the compound of formula (I) above has an endothermic peak at 233.23±4 °C in a differential scanning calorimetry curve.

In some embodiments of the present invention, the crystal form A of the compound of formula (I) above has an exothermic peak at 234.3±4 °C in a differential scanning calorimetry curve.

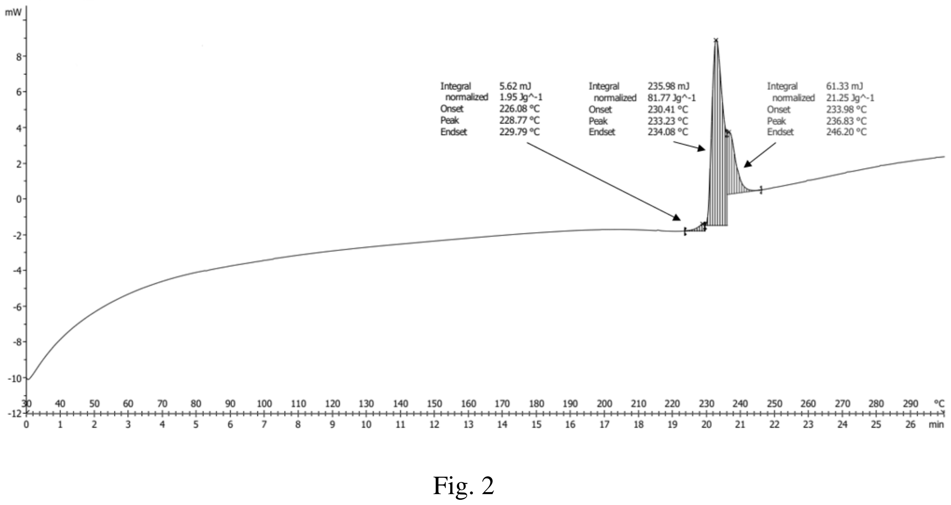

In some embodiments of the present invention, the crystal form A of the compound of formula (I) above has a differential scanning calorimetry (DSC) thermogram substantially as shown in Figure 2.

In some embodiments of the present invention, the crystal form A of the compound of formula (I) above has a weight loss of 13.50±1%, for example, a weight loss of 13.5111%, in the range of about 105-254 °C in a thermogravimetric analysis curve.

In some embodiments of the present invention, the crystal form A of the compound of formula (I) above has a weight loss of 13.50±1%, for example, a weight loss of 13.5111%, at 254.00±3 °C in a thermogravimetric analysis curve.

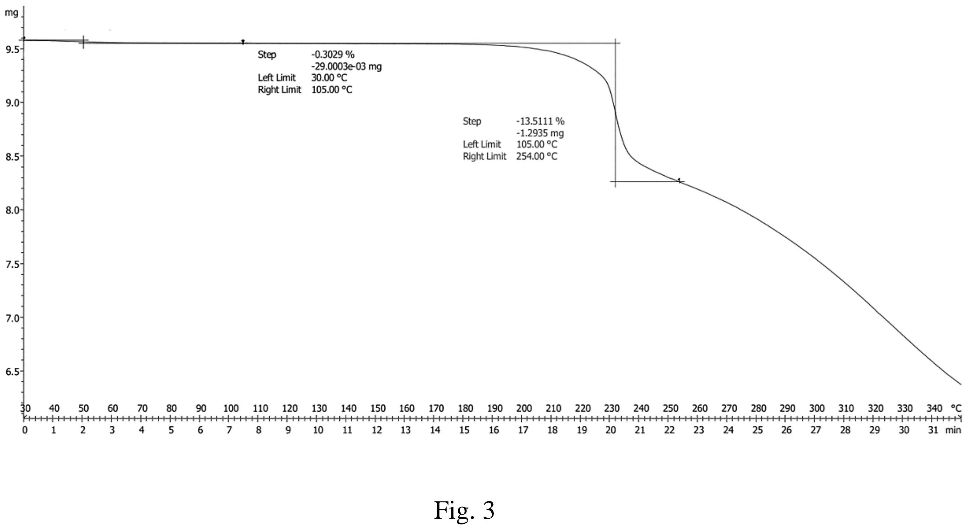

In some embodiments of the present invention, the crystal form A of the compound of formula (I) above has a thermogravimetric analysis (TGA) thermogram substantially as shown in Figure 3.

The present invention also provides a crystal form B of the compound of formula (I), which has diffraction peaks at the following 2θ angles (±0.2°): 19.19, 27.18, 13.44, and 22.55 in an X-ray powder diffraction pattern with Cu-Kα radiation.

In some embodiments of the present invention, the crystal form B of the compound of formula (I) above has diffraction peaks at the following 2θ angles (±0.2°): 26.19, 20.76, 4.42, 22.84, 19.19, 27.18, 13.44, and 22.55 in an X-ray powder diffraction pattern with Cu-Kα radiation.

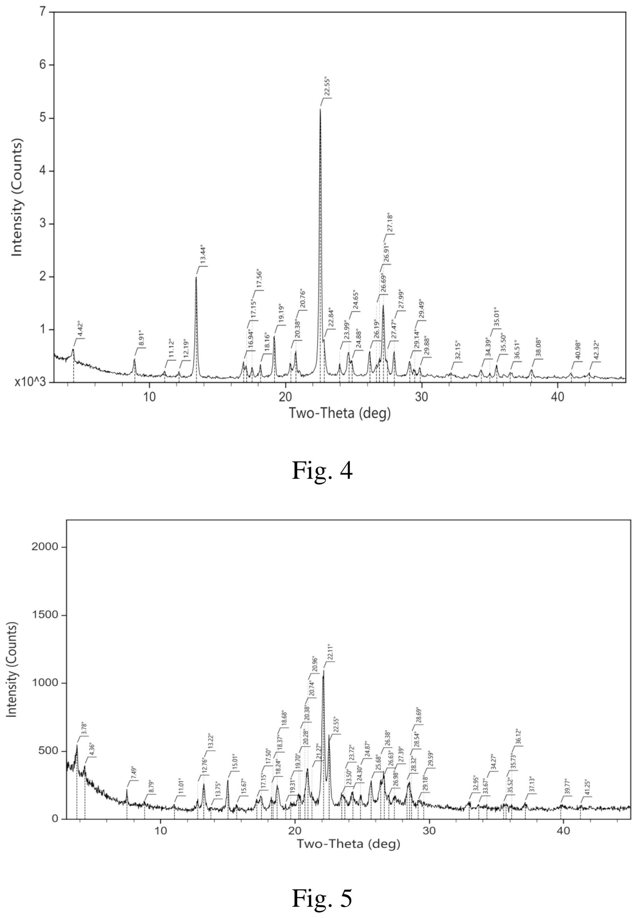

In some embodiments of the present invention, the crystal form B of the compound of formula (I) above has an XRPD pattern with Cu-Kα radiation as shown in Figure 4.

In some embodiments of the present invention, the crystal form B of the compound of formula (I) above has an XRPD pattern analysis with Cu-Kα radiation as shown in Table 3.

In some embodiments of the present invention, the crystal form B of the compound of formula (I) above is a DMF solvate of the compound of formula (I).

In some embodiments of the present invention, a ratio of the compound of formula (I) to the DMF solvent in the crystal form B of the compound of formula (I) above is 1:1.

The present invention also provides a crystal form C of the compound of formula (I), which has diffraction peaks at the following 2θ angles (±0.2°): 22.11, 22.55, 20.96, and 26.63 in an X-ray powder diffraction pattern with Cu-Kα radiation.

In some embodiments of the present invention, the crystal form C of the compound of formula (I) above has diffraction peaks at the following 2θ angles (±0.2°): 22.11, 22.55, 20.96, 26.63, 15.01, 25.68, 26.38, 28.54, 13.22, and 18.68 in an X-ray powder diffraction pattern with Cu-Kα radiation.

In some embodiments of the present invention, the crystal form C of the compound of formula (I) above has an XRPD pattern with Cu-Kα radiation as shown in Figure 5.

In some embodiments of the present invention, the crystal form C of the compound of formula (I) above has an XRPD pattern analysis with Cu-Kα radiation of as shown in Table 4.

In some embodiments of the present invention, the crystal form C of the compound of formula (I) above is a DMSO solvate of the compound of formula (I).

The present invention also provides a crystal form D of the compound of formula (I), which has diffraction peaks at the following 2θ angles (±0.2°): 22.71, 18.92, 22.96, 26.4, 24.44, 16.62, 13.15, 17.79, and 27.12 in an X-ray powder diffraction pattern with Cu-Kα radiation.

In some embodiments of the present invention, the crystal form D of the compound of formula (I) above has diffraction peaks at the following 2θ angles (±0.2°): 22.71, 18.92, 22.96, 26.4, 24.44, 16.62, 13.15, 17.79, 27.12, 24.17, 28.81, 19.95, 28.42, 24.85, 14.78, 17.36, and 29.16 in an X-ray powder diffraction pattern with Cu-Kα radiation.

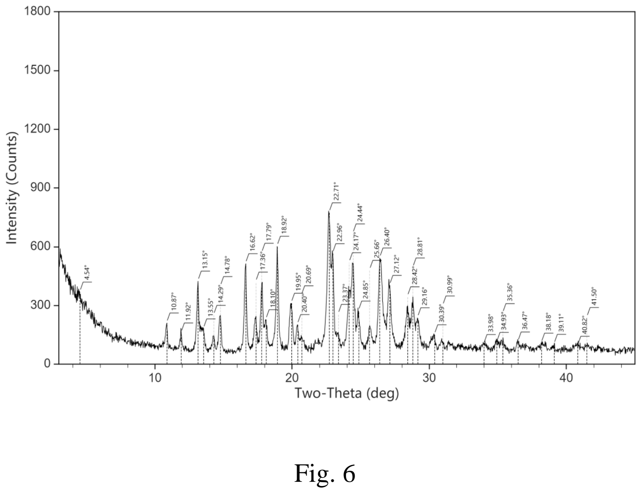

In some embodiments of the present invention, the crystal form D of the compound of formula (I) above has an XRPD pattern with Cu-Kα radiation as shown in Figure 6.

In some embodiments of the present invention, the crystal form D of the compound of formula (I) above has an XRPD pattern analysis with Cu-Kα radiation as shown in Table 5.

In some embodiments of the present invention, the crystal form D of the compound of formula (I) above is a NMP solvate of the compound of formula (I).

In some embodiments of the present invention, a ratio of the compound of formula (I) to the NMP solvent in the crystal form D of the compound of formula (I) above is 1:1.

In a second aspect, the present application provides a method for preparing the crystal form of the compound of formula (I) as described in the first aspect, which includes method I, comprising: adding the compound of formula (I) to organic solvent A to dissolve, and then adding the resulting solution to organic solvent B to precipitate crystals.

The organic solvent A is selected from one or more of DMF (N,N-dimethylformamide), DMSO (dimethyl sulfoxide), and NMP (N-methylpyrrolidone), and the organic solvent B is selected from one or more of water, methanol, ethanol, MTBE, toluene, and dichloromethane.

In some embodiments of the present invention, when the compound of formula (I) is the crystal form A, in the method I, the organic solvent A is DMF, and the organic solvent B is selected from one or two of water and ethanol; or the organic solvent A is DMSO, and the organic solvent B is selected from one or two of toluene and methanol.

In some embodiments of the present invention, when the compound of formula (I) is the crystal form B, in the method I, the organic solvent A is DMF, and the organic solvent B is selected from one or two of MTBE and toluene.

In some embodiments of the present invention, when the compound of formula (I) is the crystal form D, in the method I, the organic solvent A is NMP, and the organic solvent B is selected from one or more of water, dichloromethane, and MTBE.

The method for preparing the crystal form of the compound of formula (I) includes method II, comprising: adding the compound of formula (I) to organic solvent C to dissolve, and then adding organic solvent D to precipitate crystals.

The organic solvent C is selected from one or more of DMF, DMSO, and NMP, and the organic solvent D is selected from one or more of acetone, ethylene glycol methyl ether, water, dichloromethane, ethyl acetate, MTBE, toluene, tetrahydrofuran, dioxane, ethanol, trifluoroethanol, acetonitrile, ethylene glycol dimethyl ether, isopropanol, and butanone.

In some embodiments of the present invention, when the compound of formula (I) is the crystal form A, in the method II, the organic solvent C is DMF, and the organic solvent D is selected from one or more of acetone, ethylene glycol methyl ether, water, and dichloromethane; or the organic solvent C is DMSO, and the organic solvent D is selected from one or more of ethanol, trifluoroethanol, ethylene glycol methyl ether, acetonitrile, dioxane, ethylene glycol dimethyl ether, and butanone; or the organic solvent C is NMP, and the organic solvent D is selected from one or more of water, acetonitrile, dichloromethane, and ethylene glycol dimethyl ether.

In some embodiments of the present invention, when the compound of formula (I) is the crystal form B, in the method II, the organic solvent C is DMF, and the organic solvent D is selected from one or more of ethyl acetate, MTBE, toluene, tetrahydrofuran, dioxane, and butanone.

In some embodiments of the present invention, when the compound of formula (I) is the crystal form C, in the method II, the organic solvent C is DMSO, and the organic solvent D is selected from one or more of ethyl acetate, water, and toluene.

In some embodiments of the present invention, when the compound of formula (I) is the crystal form D, in the method II, the organic solvent C is NMP, and the organic solvent D is selected from one or more of isopropanol, MTBE, toluene, tetrahydrofuran, and butanone.

The method for preparing the crystal form of the compound of formula (I) includes method III, comprising: suspending the compound of formula (I) in organic solvent E, slurrying and filtering to obtain crystals.

The organic solvent E is selected from one or more of methanol, ethanol, isopropanol, ethyl acetate, n-heptane, MTBE, ethylene glycol methyl ether, water, acetonitrile, toluene, dichloromethane, tetrahydrofuran, dioxane, ethylene glycol dimethyl ether, butanone, dimethylformamide, dimethyl sulfoxide, N-methylpyrrolidone, and acetone.

In some embodiments of the present invention, when the compound of formula (I) is the crystal form A, in the method III, the organic solvent E is selected from one or more of methanol, ethanol, isopropanol, ethyl acetate, n-heptane, MTBE, ethylene glycol methyl ether, water, acetonitrile, toluene, dichloromethane, tetrahydrofuran, dioxane, ethylene glycol dimethyl ether, and butanone.

In some embodiments of the present invention, when the compound of formula (I) is the crystal form B, in the method III, the organic solvent E is DMF.

In some embodiments of the present invention, when the compound of formula (I) is the crystal form C, in the method III, the organic solvent E is DMSO.

In some embodiments of the present invention, when the compound of formula (I) is the crystal form D, in the method III, the organic solvent E is NMP.

In a third aspect, the present application provides a crystalline composition comprising the crystal form of the compound of formula (I) as described in the first aspect. Preferably, in the crystalline composition, the crystal form of the compound of formula (I) is selected from one or more of the crystal form A, the crystal form B, the crystal form C, and the crystal form D.

In some embodiments of the present application, the crystal form of the compound of formula (I) accounts for 10% or more, 20% or more, 30% or more, 40% or more, 50% or more, 60% or more, 70% or more, 80% or more, 90% or more, 91% or more, 92% or more, 93% or more, 94% or more, 95% or more, 96% or more, 97% or more, 98% or more, 99% or more, or 99.5% or more, by weight of the crystalline composition.

Preferably, the crystal form of the compound of formula (I) accounts for 50% or more, 60% or more, 70% or more, 80% or more, 90% or more, 91% or more, 92% or more, 93% or more, 94% or more, 95% or more, 96% or more, 97% or more, 98% or more, 99% or more, or 99.5% or more, by weight of the crystalline composition.

In some embodiments of the present application, the crystalline composition comprises the crystal form A of the compound of formula (I).

In some embodiments of the present application, the crystal form A accounts for 10% or more, 20% or more, 30% or more, 40% or more, 50% or more, 60% or more, 70% or more, 80% or more, 90% or more, 91% or more, 92% or more, 93% or more, 94% or more, 95% or more, 96% or more, 97% or more, 98% or more, 99% or more, or 99.5% or more, by weight of the crystalline composition.

Preferably, the crystal form A accounts for 50% or more, 60% or more, 70% or more, 80% or more, 90% or more, 91% or more, 92% or more, 93% or more, 94% or more, 95% or more, 96% or more, 97% or more, 98% or more, 99% or more, or 99.5% or more, by weight of the crystalline composition.

In a fourth aspect, the present application provides a pharmaceutical composition comprising the crystal form of the compound of formula (I) and optionally a pharmaceutically acceptable carrier. Preferably, in the pharmaceutical composition, the crystal form of the compound of formula (I) is selected from one or more of the crystal form A, the crystal form B, the crystal form C, and the crystal form D.

In some embodiments of the present application, the pharmaceutical composition comprises the crystal form A of the compound represented by formula (I) and optionally a pharmaceutically acceptable carrier.

In a fifth aspect, the present application provides a pharmaceutical composition comprising the crystalline composition as described in the third aspect and optionally a pharmaceutically acceptable carrier.

In a sixth aspect, the present application provides a use of the crystal form of the compound of formula (I) as described in the first aspect, the crystalline composition as described in the third aspect, or the pharmaceutical composition as described in the fourth aspect as a medicament or in the preparation of a medicament.

In some embodiments of the present application, the medicament is used to prevent and/or treat ENPP1-mediated diseases.

In some embodiments of the present application, the medicament is used to prevent and/or treat cancers or tumour-related diseases, or cardiovascular diseases.

In some embodiments of the present application, the pharmaceutical composition further comprises one, two or more additional drugs for treating cancers or tumours.

In some embodiments of the present application, the pharmaceutical composition is used in combination with one, two or more additional drugs for treating cancers or tumours or a treatment means.

Preferably, the anti-tumour drug is a chemotherapeutic drug, a targeted therapeutic drug or an immunotherapeutic drug. Preferably, the drug for preventing and/or treating cancers or tumours includes, but is not limited to: cell signal transduction inhibitors, chlorambucil, melphalan, cyclophosphamide, ifosfamide, busulfan, carmustine, lomustine, streptozotocin, cisplatin, carboplatin, oxaliplatin, dacarbazine, temozolomide, procarbazine, methotrexate, fluorouracil, cytarabine, gemcitabine, mercaptopurine, fludarabine, vinblastine, vincristine, vinorelbine, paclitaxel, docetaxel, topotecan, irinotecan, etoposide, trabectedin, dactinomycin, doxorubicin, epirubicin, daunomycin, mitoxantrone, bleomycin, mitomycin C, ixabepilone, tamoxifen, flutamide, gonadorelin analogs, megestrol, prednisone, dexamethasone, methylprednisolone, thalidomide, interferon alpha, leucovorin, sirolimus, temsirolimus, everolimus, afatinib, alisertib, amuvatinib, apatinib, axitinib, bortezomib, bosutinib, brivanib, cabozantinib, cediranib, crenolanib, crizotinib, dabrafenib, dacomitinib, danusertib, dasatinib, dovitinib, erlotinib, foretinib , ganetespib, gefitinib, ibrutinib, icotinib, imatinib, iniparib, lapatinib, lenvatinib, linifanib, linsitinib, masitinib, momelotinib, motesanib, neratinib, nilotinib, niraparib, oprozomib, olaparib, pazopanib, pictilisib, ponatinib, quizartinib, regorafenib, rigosertib, rucaparib, ruxolitinib, saracatinib, saridegib, sorafenib, sunitinib, telatinib, tivantinib, tivozanib, tofacitinib, trametinib, vandetanib, veliparib, vemurafenib, erivedge, volasertib, alemtuzumab, bevacizumab, brentuximab vedotin, catumaxomab, cetuximab, denosumab, gemtuzumab, ipilimumab, nimotuzumab, ofatumumab, panitumumab, rituximab, tositumomab, trastuzumab, PI3K inhibitors, CSF1R inhibitors, A2A and/or A2B receptor antagonists, IDO inhibitors, anti-PD-1 antibodies, anti-PD-L1 antibodies, LAG3 antibodies, TIM-3 antibodies, TIGIT antibodies, CD47 antibodies, CLAUDIN 18.2 antibodies, anti-CTLA-4 antibodies or any combination thereof. Preferably, the treatment means is radiotherapy or surgery.

In some embodiments of the present application, the medicament is used to prevent and/or treat skin cancers, bladder cancers, ovarian cancers, breast cancers, gastric cancers, pancreatic cancers, prostate cancers, colon cancers, lung cancers, bone cancers, brain cancers, neurocytoma, rectal cancers, colon cancers, familial adenomatous polyposis, hereditary non-polyposis colorectal cancers, esophageal cancers, lip cancers, laryngeal cancers, hypopharyngeal cancers, tongue cancers, salivary gland cancers, adenocarcinoma, medullary thyroid carcinomas, papillary thyroid cancers, kidney cancers, renal parenchymal cancers, cervical cancers, corpus uteri cancers, endometrial cancers, choriocarcinoma, testicular cancers, urinary cancers, melanoma, brain tumours such as glioblastoma, astrocytoma, meningioma, medulloblastoma and peripheral neuroectodermal tumours, Hodgkin's lymphoma, non-Hodgkin's lymphoma, Burkitt's lymphoma, leukemia, acute lymphoblastic leukemia (ALL), chronic lymphoblastic leukemia (CLL), acute myeloid leukemia (AML), chronic myeloid leukemia (CML), adult T-cell leukemia lymphoma, diffuse large B-cell lymphoma (DLBCL), hepatocellular carcinoma, gallbladder cancers, bronchogenic carcinoma, small cell lung cancers, non-small cell lung cancers, multiple myeloma, basal cell tumours, teratoma, retinoblastoma, choroidal melanoma, seminoma, rhabdomyosarcoma, craniopharyngioma, osteosarcoma, chondrosarcoma, myosarcoma, liposarcoma, fibrosarcoma, Ewing's sarcoma and plasmacytoma.

In some embodiments of the present application, the cardiovascular disease to be treated and/or prevented is myocardial infarction (MI), heart failure (HF), cardiac trauma, abnormal scar formation in cardiac tissue, cardiomyopathy, myocardial cell death, enhanced cardiac repair, and the release of pro-inflammatory molecules in myocardial cells.

### Definitions and Description

Unless otherwise specified, the following terms and phrases used herein are intended to have the following meanings. A particular phrase or term should not be considered indefinite or unclear without a specific definition, but rather should be understood according to its ordinary meaning.

The term "the crystal form of the compound of formula (I)" mentioned in the present application refers to the compound of formula (I) in crystalline form, including an anhydrous and solvent-free form, a hydrate form and a solvate form of the compound of formula (I). The crystal form is preferably an anhydrous and solvent-free form or a hydrate form; more preferably an anhydrous and solvent-free form.

The term "solvate" refers to an association formed by a stoichiometric or non-stoichiometric amount of solvent molecules with the compound of formula (I) of the present application, including an association containing both water molecules and one or more other solvent molecules, and an association only containing one or more other solvent molecules.

The term "hydrate" refers to an association formed by a stoichiometric or non-stoichiometric amount of water molecules with the compound of formula (I) of the present application.

The "anhydrous and solvent-free form" means that there are no water molecules or solvent molecules in the unit cell or crystal lattice, or the water molecules or solvent molecules coexist with the compound of formula (I) in a non-intermolecular force-binding manner, such as in an adsorption manner.

The term "crystalline composition" refers to a solid form comprising the crystal form A mentioned in the present application. Furthermore, in addition to the crystal form according to the present application, the crystalline composition may optionally comprise other crystal form(s) or other amorphous form(s) of the compound of formula (I) or a salt thereof, or impurities other than these substances. Those skilled in the art should understand that the sum of the content of each component in the crystalline composition should be 100%.

The "room temperature" is the room temperature in the conventional sense in the art, and is generally 10-30 °C, preferably 25 °C±5 °C.

In the X-ray powder diffraction pattern, the term "substantially" or "substantially as shown in figure" refers to a substantially pure crystal form, of which at least 50%, or at least 60%, or at least 70%, or at least 80%, or at least 90%, or at least 95%, or at least 96%, or at least 97%, or at least 98%, or at least 99% of the peaks in the X-ray powder diffraction pattern appear in the given pattern. Further, when the content of a certain crystal form in a product gradually decreases, some of the diffraction peaks attributed to said crystal form in its X-ray powder diffraction pattern may become fewer due to a factor of the detection sensitivity of the instrument. Furthermore, for any given crystal form, there may be slight errors in the position of the peaks, which is also well known in the field of crystallography. For example, the position of the peaks may shift due to changes in temperature, sample movement, instrument calibration or the like during sample analysis, and the measurement error of the 2θ values is sometimes about ±0.3°, typically about ±0.2°. Therefore, this error should be taken into account when determining the structure of each crystal form, and the term "substantially" or "substantially as shown in figure" is also intended to cover such variations in the position of diffraction peaks, which refers to ±0.3°, preferably ±0.2°, and further preferably ±0.1°.

In the DSC thermogram, the term "substantially" or "substantially as shown in figure" means that for the same crystal form of the same compound, errors in a thermal transition onset temperature, a peak temperature of an endothermic peak, a peak temperature of an exothermic peak, a melting point and so on is typically within about 8 °C, usually within about 5 °C, and usually within about 3 °C in consecutive analyses. When describing a compound as having a given thermal transition onset temperature, peak temperature of endothermic peak, peak temperature of exothermic peak, melting point and so on, it refers to the temperature ± 5 °C.

The term "prevent", "prevention" and "preventing" as used herein means that when used for a disease or condition (e.g., a viral disease), a compound or a drug (e.g., the combination product as sought protection in the present application) can reduce the frequency or delay the onset of symptoms of the medical condition in a subject compared to a subject to which the compound or the drug is not administered.

The term "treat", "treatment" and "treating" as used herein refers to alleviating, relieving, or ameliorating symptoms of a disease or disorder, ameliorating symptoms caused by underlying metabolism, suppressing a disease or disorder, for example, arresting the progression of a disease or disorder, alleviating a disease or disorder, causing regression of a disease or disorder, alleviating a condition caused by a disease or disorder, or arresting symptoms of a disease or disorder.

The pharmaceutical composition of the present application can be prepared by conventional methods in the art.

In the context of the present application, the term "pharmaceutically acceptable carrier" or "excipient" or "pharmaceutically acceptable excipient" refers to those excipients that have no obvious irritation to an organism, and do not impair the biological activity and performances of an active compound. The term "pharmaceutically acceptable excipient " includes solvents, propellants, solubilizers, co-solvents, emulsifiers, colorants, adhesives, disintegrants, fillers, lubricants, wetting agents, osmotic pressure regulators, stabilizers, glidants, flavoring agents, preservatives, suspending agents, coating materials, fragrances, anti-adhesives, antioxidants, chelating agents, penetration enhancers, pH regulators, buffers, plasticizers, surfactants, foaming agents, defoaming agents, thickeners, inclusion agents, humectants, absorbents, diluents, flocculants and deflocculants, filter aids, release retardants, etc. Those skilled in the art can select specific pharmaceutically acceptable excipients according to actual needs. The knowledge about excipients is well known to those skilled in the art, and for example, reference may be made to "Pharmaceutics" (edited by Fude Cui, 5th edition, The People's Health Press, 2003).

The wording "comprise" or "include" and English variations thereof, such as "comprises", "comprising", "includes", or "including" should be construed in an open and non-exclusive sense, i.e., "including but not limited to".

Within the scope of the present application, various options of any feature can be combined with various options of other features to form many different embodiments. The present application is intended to include all possible embodiments consisting of various options of all technical features.

The intermediate compounds in the present application can be prepared by a variety of synthetic methods well known to those skilled in the art, including specific embodiments as listed below, embodiments formed by their combination with other chemical synthesis methods, and equivalent alternatives well known to those skilled in the art. Preferred embodiments include, but are not limited to, the examples of the present application.

The chemical reactions of the specific embodiments of the present application are carried out in suitable solvents which are adapted to the chemical changes of the present application and the required reagents or materials thereof. In order to obtain the compounds of the present application, it is sometimes necessary for those skilled in the art to modify or select synthesis steps or reaction schemes on the basis of the existing embodiments.

The present application will be described in detail below by way of examples, which are not intended to be any limitation to the present application.

All reagents and solvents used in the present application are commercially available, and can be used without further purification.

### Technical Effects

The compound of formula (I) of the present invention has good PK properties and oral bioavailability, a stable crystal form, and a good druggability potential. The compound of formula (I) exhibits a good inhibitory activity against ENPP1. The compound of formula (I) has a good pharmacokinetic indicator.

The crystal form of the compound of formula (I) and the specific crystal forms thereof provided by the present invention have one or more of the following beneficial effects: (1) the crystal form of the compound of formula (I) has good characteristics, making it easy to weigh, transfer, separate, purify and store; (2) the crystal form of the compound of formula (I) and the specific crystal forms thereof have good crystallinity; (3) the crystal form of the compound of formula (I) and the specific crystal forms thereof have excellent operability, such as, ease of purification, filtration and separation, especially the crystal form A, which is easy to prepare in a high yield; and (4) the preferred crystal forms have good physical and chemical stability, especially the crystal form A, which has high purity and can be used directly as an active pharmaceutical ingredient, offering a good prospect for medicinal use.

### 1. Instruments and analytical methods

### 1.1. X-Ray Powder Diffraction (X-ray powder diffractometer, XRPD)

Instrument Model: D8 Advance
Test Conditions: Chinese Pharmacopoeia, Part IV, General Rule 0451,
X-ray Generator: copper target
Tube Voltage: 40KV
Scattering Slit: 0.6mm
Detector Slit: 10.5 mm
Anti-scatter Slit: 2.5°
Scanning Range: 3-50°
Step Width: 0.1s/step
Step Size: 0.02°

### 1.2. Differential Scanning Calorimetry (Differential Scanning Calorimeter, DSC)

Instrument Model: DSC 3
Test Conditions: starting temperature 30 °C, heating to 300 °C at a heating rate of 10 °C/min; purge gas flow rate: 50 mL/min; drying gas flow rate: 150 mL/min; purge gas: N₂; crucible: aluminum crucible.

### 1.3. Thermal Gravimetric Analysis (Thermal Gravimetric Analyzer, TGA)

Instrument Model: TGA 2
Test Conditions: starting temperature, 30 °C, heating to 350 °C at a heating rate of 10 °C/min; balance protection gas flow rate: 20 mL/min; reaction gas flow rate: 50 mL/min; purge gas: N₂; crucible: alumina crucible.

### 1.4. Dynamic Vapor Sorption (DVS)

Instrument Model: DVS Intrinsic
Test method: 25 °C 0%RH-90%RH-0%RH; referring to Chinese Pharmacopoeia, Part IV, General Rule 0103
Balanced dm/dt: 0.002
RH (%) measurement gradient: 10% per step
RH (%) measurement gradient range: 0% ~ 90%

Description of hygroscopic characteristics and definition of hygroscopic weight gain:

| Hygroscopicity characteristics | Weight gain ratio |
|---|---|
| Extreme hygroscopicity | Hygroscopic weight gain of no less than 15% |
| hygroscopicity | Hygroscopic weight gain of less than 15% but no less than 2% |
| Slight hygroscopicity | Hygroscopic weight gain of less than 2% but no less than 0.2% |
| No or almost no hygroscopicity | Hygroscopic weight gain of less than 0.2% |

### 1.5 Stability Test of Crystal Form in Water and Biological Medium

The preparation process of the biological medium was shown in the table below. A crystalline sample was added to the biological medium and water, respectively, and shaken at a constant temperature of 37 °C for 24 hours. Sample was taken at 1 hour, 4 hours and 24 hours, respectively. The sampled solution was filtered using a 0.22 µm water-based filter membrane. Some samples with higher concentrations were appropriately diluted with a diluent. The signal peak area of the solution was measured by HPLC. Finally, the concentration of the compound in the solution was calculated based on the peak area, the HPLC standard curve of the raw material and the dilution factor. In addition, samples were taken at different time points to test their pH values, and the remaining solids were subjected to XRPD testing.

| **Biological medium** | **Preparation process** |
|---|---|
| FaSSIF | 1.04255g FaSSIF solution concentrate was weighed and diluted with an appropriate amount of pure water, and then 56.23 mg FaSSIF/FeSSIF/FaSSGF powder was added, and diluted with pure water to a final volume of 25 mL. The pH was 6.5. |
| FeSSIF | 2.03528 g FeSSIF solution concentrate was weighed and diluted with an appropriate amount of pure water, and then 280.36 mg FaSSIF/FeSSIF/FaSSGF powder was added, and diluted with pure water to a final volume of 25 mL. The pH was 5.0. |
| FaSSGF | 3.6782 g FaSSGF solution concentrate was weighed and diluted with an appropriate amount of pure water, and then 6.02 mg FaSSIF/FeSSIF/FaSSGF powder was added, and diluted with pure water to a final volume of 100 mL. The pH was 1.6. |

| | |
|---|---|
| Notes: FaSSIF (Fasted State Simulated Intestinal Fluid): simulates the intestinal fluid in the small intestine of humans in a hungry state before a meal; FeSSIF (Fed State Simulated Intestinal Fluid): simulates the intestinal fluid in the small intestine of humans in a satiated state after a meal; and FaSSGF (Fasted State Simulated Gastric Fluid): simulates the gastric fluid in the empty stomach of humans in a hungry state. | |

### BRIEF DESCRIPTION OF THE DRAWINGS

Figure 1 is an XRPD pattern of the crystal form A of the compound of formula (I) as prepared in Example 2.
Figure 2 is a DSC thermogram of the crystal form A of the compound of formula (I).
Figure 3 is a TGA thermogram of the crystal form A of the compound of formula (I).
Figure 4 is an XRPD pattern of the crystal form B of the compound of formula (I) as prepared in Example 4.
Figure 5 is an XRPD pattern of the crystal form C of the compound of formula (I) as prepared in Example 5.
Figure 6 is an XRPD pattern of the crystal form D of the compound of formula (I) as prepared in Example 6.
Figure 7 is an XRPD pattern of the crystal form A of the compound of formula (I) as prepared in Example 3.
Figure 8 shows cardiac function indicators and body weight changes in a mouse model of acute myocardial ischemia treated with the compound of formula (I). Panel (A): left ventricular ejection fraction in mice. Panel (B): left ventricular fractional shortening in mice. Panel (C): body weight changes in mice.
Figure 9 shows changes in myocardial fibrosis in a mouse model of acute myocardial ischemia treated with the compound of formula (I). Panel (D): Masson-stained pathological sections of mouse heart tissue. Panel (E) degree of myocardial fibrosis in mice.
Figure 10 shows cardiac function indicators and body weight changes in a rat model of acute myocardial ischemia treated with the compound of formula (I). Panel (a): left ventricular fractional shortening in rats. Panel (b): left ventricular ejection fraction in rats. Panel (c): body weight changes in rats.
Figure 11 shows changes in myocardial fibrosis in a rat model of acute myocardial ischemia treated with the compound of formula (I). Panel (d): Masson-stained pathological sections of rat heart tissue. Panel (e): degree of myocardial fibrosis in rats.
Figure 12 shows a comparison of XRPD patterns of the crystal form A before and after the stability experiment.

Note: "Compound 1" in the figures refers to the compound of formula (I).

### DETAILED DESCRIPTION

In order to better understand the contents of the present invention, further description will be given below in conjunction with specific examples, but the specific examples are not intended to limit the contents of the present invention.

### Example 1: Synthesis of Compound of Formula (I)

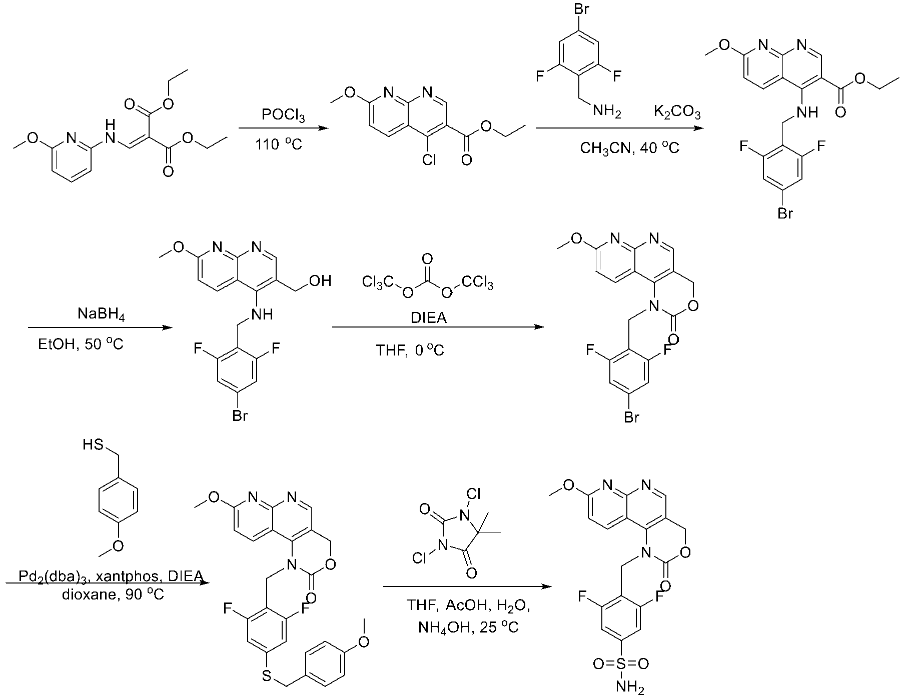

### Step 1: Preparation of ethyl 4-chloro-7-methoxy-1,8-naphthyridine-3-carboxylate

1,3-Diethyl 2-(((6-methoxypyridin-2-yl)amino)methylene)malonate (12 g, 22.7 mmol, 1 *eq*.) was dissolved in phosphorus oxychloride (60 mL), and the reaction mixture was stirred at 110 °C for 4 hours under the protection of nitrogen gas. The completion of the reaction was monitored by LCMS. The solvent was distilled off under reduced pressure. The resulting residue was dissolved in ethyl acetate (60 mL) and washed quickly with water (20 mL). The organic phase was dried over anhydrous sodium sulfate, and filtered, and the filtrate was dried by rotary evaporation. The resulting crude product was separated and purified by flash chromatography (Silica gel, DCM: EA=1: 1) to obtain the target compound (1.5 g, yield 12%). LCMS (ESI) [M+H]⁺ = 266.0.

### Step 2: Preparation of ethyl 4-((4-bromo-2,6-difluorobenzyl)amino)-7-methoxy-1,8-naphthyridine-3-carboxylate

Ethyl 4-chloro-7-methoxy-1,8-naphthyridine-3-carboxylate (3.0 g, 18.75 mmol, 1 *eq*.) was dissolved in acetonitrile (80 mL), and then potassium carbonate (5.2 g, 37.5 mmol, 2.0 *eq*.) and (4-bromo-2,6-difluorophenyl)methanamine (4.37 g, 19.69 mmol, 1.05 *eq*.) were added, and the reaction was carried out at 40 °C for 20 hours under the protection of nitrogen gas. The completion of the reaction was monitored by LCMS. The reaction mixture was concentrated, diluted with ethyl acetate, and washed with water. The organic phase was dried over anhydrous sodium sulfate, and filtered, and the filtrate was dried by rotary evaporation. The resulting crude product was separated and purified by flash chromatography (Silica gel, DCM: EA=4: 1) to obtain the target compound (3.5 g, yield 69.2%). LCMS (ESI) [M+H]⁺ = 452.0.

### Step 3: Preparation of (4-((4-bromo-2,6-difluorobenzyl)amino)-7-methoxy-1,8-naphthyridin-3-yl)methanol

Ethyl 4-((4-bromo-2,6-difluorobenzyl)amino)-7-methoxy-1,8-naphthyridine-3-carboxylate (120 mg, 22.7 mmol, 1 *eq.*) was dissolved in ethanol (10 mL), and sodium borohydride (134 mg, 5 *eq*.) was slowly added, and then the reaction mixture was stirred at 50 °C for 16 hours under the protection of nitrogen gas. The completion of the reaction was monitored by LCMS. The mixture was dried by rotary evaporation to remove the solvent. The resulting residue was quenched with a saturated sodium chloride solution (5 mL), and extracted with ethyl acetate (10 mL). The organic phase was dried over anhydrous sodium sulfate and filtered, and the filtrate was dried by rotary evaporation. The resulting crude product was separated and purified by flash chromatography (Silica gel, DCM: MeOH=2: 1) to obtain the target compound (120 mg, yield 38%). LCMS (ESI) [M+H] ⁺ = 410.0; ¹H NMR (400 MHz, CDCl₃) δ 8.43 (s, 1H), 8.35 (d, J = 9.2 Hz, 1H), 7.09 (d, J = 6.8 Hz, 2H), 6.90 (d, J = 9.2 Hz, 1H), 4.75 (s, 2H), 4.69 (s, 2H), 4.12 (s, 3H).

### Step 4: Preparation of 1-(4-bromo-2,6-difluorobenzyl)-8-methoxy-1,4-dihydro-2H-[1,3]oxazino[5,4-c][1,8]naphthyridin-2-one

(4-((4-bromo-2,6-difluorobenzyl)amino)-7-methoxy-1,8-naphthyridin-3-yl)methanol (300 mg, 0.73 mmol, 1 *eq*.) was dissolved in dichloromethane (15 mL), and *N,N*-diisopropylethylamine (473 mg, 3.66 mmol, 5 *eq.*) and triphosgene (651 mg, 2.19 mmol, 3 *eq.*) were added, and the reaction mixture was reacted at 0 °C for 2 hours.

The completion of the reaction was monitored by LCMS. The reaction mixture was washed with water, dried over anhydrous sodium sulfate, and filtered, and the filtrate was dried by rotary evaporation. The resulting crude product was separated and purified by flash silica gel column chromatography (Silica gel, MeOH: DCM=5: 95) to obtain the target compound (250 mg, yield 78.51%). LCMS (ESI) [M+H]⁺ = 436.0.

### Step 5: Preparation of 1-(2,6-difluoro-4-((4-methoxybenzyl)thio)benzyl)-8-methoxy-1,4-dihydro-2H-[1,3]oxazino[5,4-c] [1,8]naphthyridin-2-one

1-(4-bromo-2,6-difluorobenzyl)-8-methoxy-1,4-dihydro-2*H*-[1,3]oxazino[5,4-c][1,8]naphthyridin-2-one (200 mg, 0.46 mmol, 1 *eq*.) was dissolved in 1,4-dioxane (10 mL), and (4-methoxyphenyl)methanethiol (141 mg, 0.92 mmol, 2 *eq*.)*,* [5-(diphenylphosphino)-9,9-dimethyl-9*H-*xanthogen-4-yl]diphenylphosphine (53 mg, 0.09 mmol, 0.2 *eq*.)*,* tris(1,5-diphenylpenta-1,4-dien-3- one)dipalladium (84 mg, 0.09 mmol, 0.2 *eq*.) and *N,N*-diisopropylethylamine (178 mg, 1.38 mmol, 3 *eq*.) were added. The reaction mixture was reacted at 90 °C for 2 hours. The completion of the reaction was monitored by LCMS. The reaction mixture was concentrated and dried by rotary evaporation, and then was separated and purified by flash silica gel column chromatography (Silica gel, MeOH: DCM=5: 95) to obtain the target compound (110 mg, yield 47.09%). LCMS (ESI) [M+H]⁺=510.1.

### Step 6: Preparation of 3,5-difluoro-4-((8-methoxy-2-oxo-2H-[1,3]oxazino[5,4-c][1,8]naphthyridin-1(4H)-yl)methyl)benzenesulfonamide

1-(2,6-difluoro-4-((4-methoxybenzyl)thio)benzyl)-8-methoxy-1,4-dihydro-2*H-*[1,3]oxazino[5,4-c][1,8]naphthyridin-2-one (100 mg, 0.2 mmol, 1.0 *eq*.), acetic acid (83 mg, 1.37 mmol, 7.0 *eq*.), and water (50 mg, 2.75 mmol, 14.0 *eq*.) were dissolved in tetrahydrofuran (15 mL), and 1,3-dichloro-5,5-dimethylhydantoin (116 mg, 0.59 mmol, 3 *eq*.) was added at 0 °C, and then the reaction mixture was reacted at 25 °C for 1 hour. Aqueous ammonia (0.5 mL) was added and the resulting mixture was reacted at 25 °C for 10 minutes. LCMS monitoring showed that the starting materials were completely reacted. The reaction mixture was concentrated and purified by preparative HPLC to obtain the target compound (14.6 mg, yield 17.05%). LCMS (ESI) [M+H]⁺ = 437.1; ¹HNMR (400MHz, MeOD-d₄) δ 8.71 (s, 1H), 8.59 (d, J = 9.2 Hz, 1H), 7.46 (d, J = 7.6 Hz, 2H), 7.13 (d, J = 9.6 Hz, 1H), 5.57 (s, 2H), 5.30 (s, 2H), 4.12 (s, 3H).

### Example 2: Preparation of Crystal Form A of Compound of Formula (I)

To a reaction flask the compound of formula (I) as prepared in Example 1 (100 mg) and dimethyl sulfoxide (0.3 mL) were successively added, slowly heated to dissolve, and then slowly added to water (1 mL) at 80 °C. After slowly cooling to room temperature, the resulting mixture was stirred for one hour and filtered. The filter cake was washed with an appropriate amount of water, and dried under reduced pressure at 65 °C to afford the crystal form A of the compound of formula (I).

**Table 1: XRPD Pattern Analysis Data of Crystal Form A of Compound of Formula (I)**

| No. | 2θ angle (°) | Interplanar Distance (Å) | Relative Intensity (%) | No. | 2θ angle (°) | Interplanar Distance (Å) | Relative Intensity (%) |
|---|---|---|---|---|---|---|---|
| 1 | 12.763 | 6.93059 | 14.5 | 7 | 24.851 | 3.57990 | 100 |
| 2 | 13.957 | 6.34010 | 8.4 | 8 | 25.072 | 3.54894 | 79.2 |
| 3 | 15.995 | 5.53658 | 5.9 | 9 | 25.718 | 3.46127 | 63.8 |
| 4 | 16.971 | 5.22022 | 1.8 | 10 | 26.904 | 3.31126 | 11.6 |
| 5 | 18.481 | 4.79708 | 8.1 | 11 | 28.488 | 3.13064 | 8.9 |
| 6 | 20.480 | 4.33298 | 4.2 | 12 | 29.819 | 2.99386 | 8.3 |

### Example 3: Preparation of Crystal Form A of Compound of Formula (I)

To a reaction flask the compound of formula (I) as prepared in Example 1 (100 mg) was added, and then acetone (1 mL) was added. The resulting mixture was stirred for 24 hours at room temperature, suspended and slurried, and then filtered. The filter cake was dried under reduced pressure at 45 °C to afford the crystal form A of the compound of formula (I).

**Table 2: XRPD Pattern Analysis Data of Crystal Form A of Compound of Formula (I)**

| No. | 20 angle (°) | Interplanar Distance(Å) | Relative Intensity (%) | No. | 2θ angle (°) | Interplanar Distance (Å) | Relative Intensity (%) |
|---|---|---|---|---|---|---|---|
| 1 | 12.775 | 6.92376 | 20.1 | 7 | 24.871 | 3.57719 | 100 |
| 2 | 13.969 | 6.33445 | 14.0 | 8 | 25.087 | 3.54685 | 87.2 |
| 3 | 16.010 | 5.53149 | 33.4 | 9 | 25.731 | 3.45948 | 77.4 |
| 4 | 16.984 | 5.21628 | 10.8 | 10 | 26.858 | 3.31685 | 22.4 |
| 5 | 18.495 | 4.79344 | 42.1 | 11 | 28.503 | 3.12906 | 20.5 |
| 6 | 20.471 | 4.33504 | 10.6 | 12 | 29.830 | 2.99282 | 22.7 |

### Example 4: Preparation of Crystal Form B of Compound of Formula (I)

260 mg of a sample was weighed and dissolved in 10 mL of DMF at room temperature. 0.8 mL of the resulting solution was taken, and about 8 mL of methyl tert-butyl ether was added dropwise. After stirring at room temperature for a certain period of time, the system with precipitated solids was centrifuged and separated, and the solids were dried under vacuum at room temperature to afford the crystal form B.

**Table 3: XRPD Pattern Analysis Data of Crystal Form B of Compound of Formula (I)**

| No. | 20 angle (°) | Interplanar Distance (Å) | Relative Intensity (%) | No. | 2θ angle (°) | Interplanar Distance (Å) | Relative Intensity (%) |
|---|---|---|---|---|---|---|---|
| 1 | 4.42 | 19.99571 | 12.2 | 7 | 22.84 | 3.96834 | 15.6 |
| 2 | 8.91 | 9.94855 | 8.8 | 8 | 24.65 | 3.69362 | 11.1 |
| 3 | 13.44 | 6.62975 | 38.6 | 9 | 26.19 | 3.49104 | 11.4 |
| 4 | 19.19 | 4.68691 | 17.1 | 10 | 26.91 | 3.40442 | 8.9 |
| 5 | 20.76 | 4.34553 | 11.5 | 11 | 27.18 | 3.37289 | 28.3 |
| 6 | 22.55 | 4.01691 | 100 | 12 | 27.99 | 3.28216 | 11.3 |

### Example 5: Preparation of Crystal Form C of Compound of Formula (I)

260 mg of a sample was weighed and dissolved in 4 mLof DMSO at room temperature. 0.3 mL of the resulting solution was taken, and about 3 mL of toluene was added dropwise. After stirring at room temperature for a certain period of time, the system with precipitated solids was centrifuged and separated, and the solids were dried under vacuum at room temperature to afford the crystal form C.

**Table 4: XRPD Pattern Analysis Data of Crystal Form C of Compound of Formula (I)**

| No. | 2θ angle (°) | Interplanar Distance (Å) | Relative Intensity (%) | No. | 2θ angle (°) | Interplanar Distance (Å) | Relative Intensity (%) |
|---|---|---|---|---|---|---|---|
| 1 | 3.78 | 23.38464 | 52.2 | 7 | 22.11 | 4.09394 | 100 |
| 2 | 4.36 | 20.26257 | 37.6 | 8 | 22.55 | 4.01691 | 53.6 |
| 3 | 13.22 | 6.73499 | 25 | 9 | 25.68 | 3.55493 | 27.1 |
| 4 | 15.01 | 5.94803 | 27.6 | 10 | 26.38 | 3.46714 | 27 |
| 5 | 18.68 | 4.80896 | 24 | 11 | 26.63 | 3.43662 | 30 |
| 6 | 20.96 | 4.30702 | 35.3 | 12 | 28.54 | 3.22469 | 26.2 |

### Example 6: Preparation of Crystal Form D of Compound of Formula (I)

260 mg of a sample was weighed and dissolved in 10 mL of NMP at room temperature. 0.8 mL of the resulting solution was taken, and about 8 mL of methyl tert-butyl ether was added dropwise. After stirring at room temperature for a certain period of time, the system with precipitated solids was centrifuged and separated, and the solids were dried under vacuum at room temperature to afford the crystal form D.

**Table 5: XRPD Pattern Analysis Data of Crystal Form D of Compound of Formula (I)**

| No. | 2θ angle (°) | Interplanar Distance (Å) | Relative Intensity (%) | No. | 2θ angle (°) | Interplanar Distance (Å) | Relative Intensity (%) |
|---|---|---|---|---|---|---|---|
| 1 | 4.54 | 19.48264 | 47.6 | 14 | 22.71 | 3.99084 | 100 |
| 2 | 10.87 | 8.1683 | 27.2 | 15 | 22.96 | 3.94927 | 73 |
| 3 | 11.92 | 7.45824 | 20.6 | 16 | 23.37 | 3.88406 | 21 |
| 4 | 13.15 | 6.77411 | 54.9 | 17 | 24.17 | 3.76329 | 48 |
| 5 | 13.55 | 6.57377 | 23.3 | 18 | 24.44 | 3.72392 | 66.6 |
| 6 | 14.78 | 6.03976 | 32.2 | 19 | 24.85 | 3.66654 | 35.1 |
| 7 | 16.62 | 5.38486 | 66.4 | 20 | 25.66 | 3.55744 | 25.5 |
| 8 | 17.36 | 5.16242 | 31.6 | 21 | 26.4 | 3.46477 | 70.1 |
| 9 | 17.79 | 5.04222 | 53.3 | 22 | 27.12 | 3.37959 | 52 |
| 10 | 18.1 | 4.95842 | 29.4 | 23 | 28.42 | 3.23681 | 38.7 |
| 11 | 18.92 | 4.75181 | 77.9 | 24 | 28.81 | 3.19681 | 44.5 |
| 12 | 19.95 | 4.51567 | 40.2 | 25 | 29.16 | 3.16176 | 30.7 |
| 13 | 20.4 | 4.42077 | 26.1 | | | | |

### Test Example 1: Study on Hygroscopicity of Crystal Form

Experimental Instruction: DVS Intrinsic
Experimental Method: 25-35 mg of the crystal form A of the compound of formula (I) as prepared in Example 2 was placed in a DVS sample tray for testing.
Experimental Conclusion: The spectrum of the crystal form A of the compound of formula (I) showed a weight gain of ΔW=0.4461% at 25 °C and 80% humidity, indicating slight hygroscopicity. This crystal form remained unchanged after the DVS testing.

### Test Example 2: Solid Stability Study

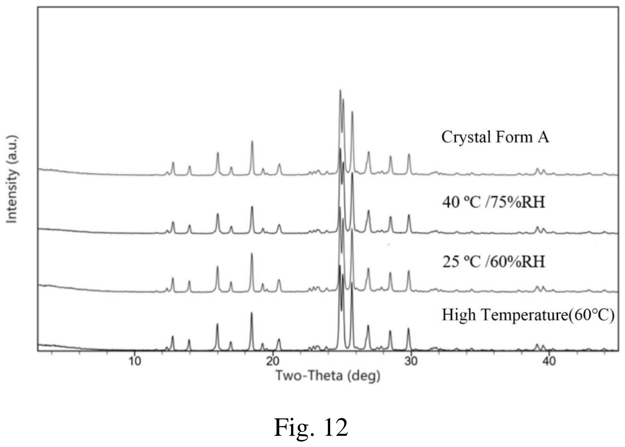

20 mg samples of the crystal form A of the compound of formula (I) as prepared in Example 2 were weighed into vials, and then left open under high temperature (60 °C), long term (25 °C/60% RH) and accelerated (40 °C/75% RH) conditions for 7 days, respectively. The samples were subjected to purity testing and X-ray powder diffraction to investigate the stability of the crystal form A of Example 2 under different conditions. The results were shown in Table 6 and Figure 12.

**Table 6: Results of Solid Stability Test**

| Initial Sample | Condition | Purity | XRPD (7 day) |
|---|---|---|---|
| | 0 day | 98% | |
| Example (Crystal Form A) | High temperature (60°C)-7d | 98.02% | Unchanged |
| | 25 °C /60%RH-7d | 98.11% | Unchanged |
| | 40 °C /75%RH -7d | 98.17% | Unchanged |

The data showed that Example 2 (i.e., the crystal form A) can maintain chemical and crystal stability in the solid stability test.

### Test Example 3: Stability Test of Crystal Form A of Compound of Formula (I) in Biological Solvent Medium

The stability test of the samples from Example 2 (i.e., the crystal form A) in a biological solvent medium was conducted in three biological media (i.e., FaSSIF, FeSSIF and FaSSGF) and water. After 24 hours, the remaining samples were subjected to X-ray powder diffraction. The results were shown in Table 7.

**Table 7: Stability Test in Biological Solvent Medium**

| Example | Initial crystal form | Medium | Initial pH | XRPD of remaining solid |
|---|---|---|---|---|
| Example 2 | Crystal form A | Water | - | Unchanged |
| | | FaSSIF | 6.5 | Unchanged |
| | | FeSSIF | 5.0 | Unchanged |
| | | FaSSGF | 1.6 | Unchanged |

The data showed that the crystal form A of Example 2 can maintain crystal stability in all biological solvent media.

### Test Example 4: ENPP1 Enzymatic Inhibition Test in Vitro

ENPP1 is a transmembrane glycoprotein capable of hydrolyzing nucleotides and derivatives with nucleotide-5'-monophosphate structures. ENPP1 is capable of hydrolyzing artificially synthesized 5'-p-nitrophenyl monophosphate (TMP-pNP) into nucleotide 5'-monophosphate and p-nitrophenol, while p-nitrophenol is a chromogenic product. The production amount of p-nitrophenol product can be directly measured by its absorbance at 405 nm, which is directly proportional to the enzyme activity.

### Experimental Procedures

100X serial gradient dilutions of compound were prepared with DMSO, starting from a stock concentration of 1 mM, and using a 4-fold serial gradient dilution to make 10 concentration points. 300 Nanoliter of compounds at gradient concentrations were transferred to a 384-well assay plate (final concentration: starting at 10 µM, 4-fold serial gradient dilution, 10 concentration points) by using Echo. First, 15 µL of 0.2 ng/uL hENPP1 enzyme (2× final concentration) prepared in an assay buffer (250 mM NaCl, 50 mM Tris, pH = 9.5) was added to each well. Then, 15 µL of 400 µM TMP-pNP (2× final concentration) prepared in the assay buffer was added. After incubation at 37 °C for 0.5 h, the OD405 nm value was read by the microplate reader. The percentage of inhibition rate was calculated using the formula: %Inhibition Rate=(OD_{high signal control} - OD_{sample well}) / (ODhigh _{signal} control - OD_{low signal control})*100. The IC₅₀ value was calculated using four-parameter fitting. The high signal control: DMSO group without inhibitors, and the low signal control: blank control group. The data of the compound of formula (I) as prepared in Example 1 were shown in Table 8.

**Table 8: Enzymatic Inhibitory Activity against ENPP1**

| Compound No. | IC₅₀ (nM) |
|---|---|
| Compound of Formula (I) | 0.05 |

### Test Example 5: Pharmacokinetic Test Using LC-MS/MS to Determine Compound Concentration in Mice

**Test principle:** The drug concentration of the target drug in plasma at different times was determined by using LC-MS/MS, and the pharmacokinetic profile of the target compound *in vivo* was plotted.

**Test method:** The test compound (i.e., the compound of formula (I) prepared in Example 1) was dissolved in DMSO to prepare a stock solution having a final concentration of 20 mg/mL. The above compound was diluted to 1 mg/mL in a solvent containing 5% DMSO (Sigma-Aldrich, SHBJ2847), 45% PEG400 (Sigma-Aldrich, BCCC0015) and 50% ddH₂O. Mice were sourced from CD-1 male mice (JH Laboratory Animal Co. LTD), with nine mice for each compound group. The mice were administered with 10 mg/kg of the compound by oral gavage (PO). Blood was collected at 0.25 hr, 0.5 hr, 1 hr, 2 hr, 4 hr, 8 hr, and 24 hr, respectively, with three samples taken at each time point, and 110 µL of whole blood (K2EDTA anticoagulation) was collected. The samples were immediately centrifuged at 2000 g for 5 minutes at 4° C, and the serum was collected and stored at - 70°C. The drug concentration in plasma was determined using a Triple-quadrupole MS system (SCIEX), including standard curve and quality control preparation, and sample preparation. Standard curve and quality control preparation: the standard curve and quality control working solution was formulated with MeOH:H₂O (1:1), and 3 µL of the above working solution was added to 57 µL of blank plasma. Sample preparation: 30 µL of plasma sample was added to 200 µL of internal standard solution (Propranolol, 40 ng/mL), mixed for 1 minute, and then centrifuged at 5800 rpm for 10 minutes, and 100 µL of supernatant was transferred to a new plate for sample analysis. The chromatographic conditions were optimized for each sample, including mobile phase composition, elution gradient conditions, flow rate, and retention time. The chromatographic column was Waters BEH C18 (2.1×50 mm, 1.7 m), and the injection volume was 1 µL. Mass spectrometry was performed using an electrospray ionization source (TuREo spray). In the positive ion detection mode, the multichannel reaction monitoring (MRM) mode was selected for secondary mass spectrometry analysis. Based on the drug concentration-time data, pharmacokinetic parameters were calculated using WinNonlin 8.2 software according to a non-compartmental model, including peak concentration Cₘₐₓ, peak time Tₘₐₓ, area under the drug-time curve AUC, and elimination half-life t½. The AUC was calculated using the linear trapezoidal method (linear up log down).

**Table 9: Pharmacokinetic Parameters**

| | IV, 2mpk | | | | PO, 10mpk | | | | |
|---|---|---|---|---|---|---|---|---|---|
| Compound No. | T_{1/2,} h | AUC₀₋₂₄ₕ, h*ng/mL | CL, L/h/kg | Vss, L/kg | T_{1/2}, h | Cₘₐₓ, ng/mL | Tₘₐₓ, h | AUC₀₋₂₄ₕ, h*ng/mL | F% |
| Compound of Formula (I) | 5.35 | 10021 | 0.192 | 1.08 | 3.5 | 8080 | 2 | 47811 | 92.6 |

The test results showed that the compound of formula (I) of the present invention had good pharmacokinetics *in vivo* and had a druggability potential.

### Test Example 6: Pharmacodynamic Evaluation of Compound of Formula (I) in Mouse Model of Pancreatic Cancer Pan02

**1. Experimental Objective:** The *in vivo* efficacy of the test drug in the mouse subcutaneous homograft tumour model of pancreatic cancer Pan02 cells was evaluated.
**2. Experimental Methods**
   **2.1 Cell Culture**
      Mouse pancreatic cancer Pan02 cells were cultured in monolayer *in vitro* in DMEM medium supplemented with 10% fetal bovine serum, 0.01 mg/mL sinsulin, 1% penicillin/streptomycin/amphotericin B in an incubator at 37 °C with 5% CO₂. When the cell saturation was 80%-90% and the number reached the requirement, the cells were harvested, counted, and inoculated.
   **2.2 Animals**
      C57BL/6 mice, female, 6-8 weeks old, weighing 18-22 g. The mice were provided by Shanghai Xipu-Bikai Laboratory Animal Co., Ltd., or other suppliers.
   **2.3 Tumour Inoculation and Animal Grouping and administration**
      0.1 Milliliter of Pan02 cells (5×10⁶ cells) were inoculated subcutaneously into the right hind back of each mouse, and animal grouping and administration began when the average tumour volume reached about 100-200 mm³. Animals were weighed and tumour volumes were measured before administration. Mice were randomly divided into groups based on the tumour volume, with 8 mice in each group. The control group was given the vehicle: (5% DMSO + 45% PEG400 + 50% H₂O (1% CMC-Na)), while the treatment group was administered by an oral gavage at a dose of 20 mg/kg with a dosing volume of 10 µL/g body weight twice daily. Animals were observed daily for health status. If the tumour volume exceeded 3,000 mm³, or if there were signs of severe illness, pain, body weight loss greater than 20%, or continuous deterioration in animals, euthanasia was performed.
   **2.4 Tumour Growth Inhibition (TGI)**
      The tumour diameter was measured twice a week using a vernier calliper. Tumor volume was calculated using the formula: V=0.5*a*×*b*², where a and b represented the long and short diameters of the tumour, respectively. The tumour growth inhibition TGI (%) of the compound was calculated as follows: TGI (%) = [(1-(average tumour volume of the treatment group after administration - average tumour volume of the treatment group before administration))/(average tumour volume of the vehicle control group before administration - average tumour volume of the vehicle control group after administration)]×100%. Data analysis was performed using a T-test for comparison between the two groups, and p<0.05 was considered to be a significant difference.
**3. Experimental Results**
   The compound of formula (I) as prepared in Example 1 of the present application showed activity in the mouse model of pancreatic cancer Pan02. After administration for 16 days, the compound of formula (I) of the present invention significantly inhibited the growth of the tumour, with a TGI of 43.4%, which had statistically significant difference compared to the control group (p<0.05), and had no inhibitory effect on the body weight of the mice, indicating good safety. The crystal form of the compound of formula (I) (for example, the crystal form A of the compound of formula (I)) had substantially the same efficacies.

### Test Example 7: Pharmacodynamic Evaluation of Compound of Formula (I) in Mouse Myocardial Ischemia Model

### 1. Experimental Objective

This study aimed to evaluate the pharmacodynamic effect of the compound of formula (I) in a C57 BL/6 mouse model of myocardial ischemia.

### 2. Experimental Method

After the animals had acclimated to the facility for one week, they were divided into four groups based on their body weights on the day before surgery, with 6 animals in the sham group (Sham) and 8 animals in the model group (MI). On the next day, the animals underwent myocardial ischemia surgery for modeling (the mice in the Sham group only underwent thoracotomy and threading without ligation). The model was established by ligation of the left anterior descending coronary artery (LAD). After model establishment, the mice were administered penicillin (20,000 units/mouse, i.m.) for 3 days, and disinfected to prevent infection.

Each group of animals was administered the corresponding compounds one hour before surgery. The control group (Vehicle) was administered the vehicle: 5% DMSO+45% PEG400+50% H₂O (1% CMC-Na). The experimental groups were administered by oral gavage at doses of 4 mg/kg and 20 mg/kg, respectively, twice daily for 28 consecutive days. The body weights of the animals were monitored daily after administration, and echocardiographic imaging indicators were measured on days 1, 7 and 28. The primary indicators of echocardiographic evaluation were left ventricular fractional shortening (FS) and left ventricular ejection fraction (LVEF). After the echocardiographic measurement on day 28, heart tissues were collected in a tissue fixative for pathological analysis by Masson's trichrome staining.

### 3. Experimental Results

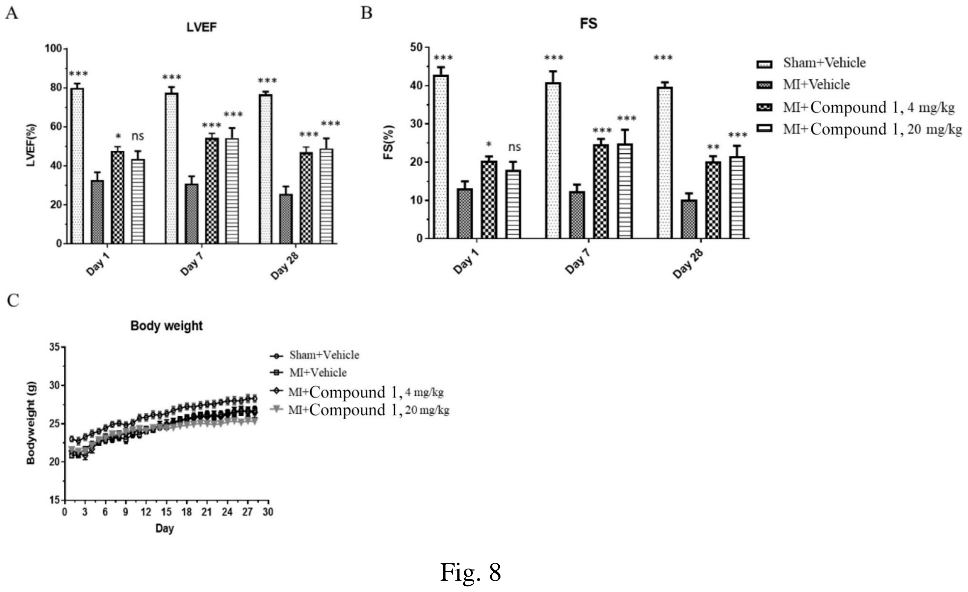

The compound of formula (I) in Example 1 of the present invention showed good cardiac function-improving activity in the mouse model of myocardial infarction. The compound of formula (I) of the present invention had a protective effect on cardiac function after pre-administration one day before modeling. As shown in Figure 8, on day 1, compared with the vehicle control group, 4 mpk and 20 mpk dosage groups increased the left ventricular ejection fraction (LVEF) of the mice by 15% and 10%, respectively (panel A), and increased the left ventricular fractional shortening (FS) of the mice by 7% and 5%, respectively (panel B). On day 7, the left ventricular ejection fraction (LVEF) of the mice was increased by 23.4% and 23.3% respectively, and the left ventricular fractional shortening (FS) of the mice was increased by 12.2% and 12.3% respectively. On day 28, the LVEF and FS of the vehicle control group further deteriorated, while the compound of formula (I) further maintained the LVEF and FS in the left ventricle of the mice without any significant effect on body weight (panel C). Statistical significance was analyzed using two-way ANOVA/one-way ANOVA/t-test, with p < 0.05 indicating a significant difference.

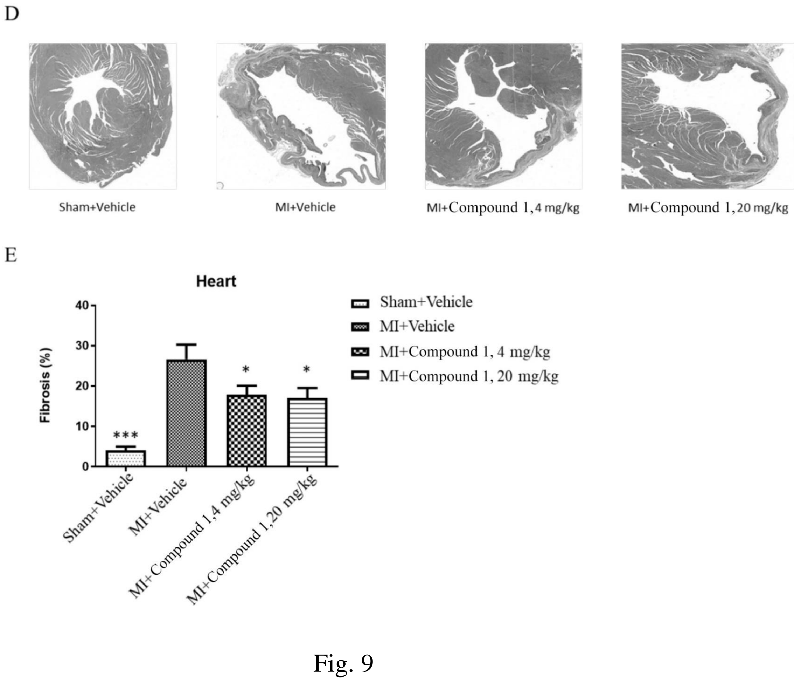

The compound of formula (I) in Example 1 of the present invention showed an improving effect on myocardial fibrosis in mice with myocardial infarction. As shown in Figure 9, panel D showed an electron micrograph of Masson-stained pathological sections of mouse heart tissue. Panel E showed statistical results of the degree of fibrosis in mouse heart tissue. As shown in the figure, in the Sham group, red myocardial fibers in the mice were arranged neatly, and blue collagen fibers were rarely seen. In the control group of the MI model, myocardial cells were necrotic, myocardial tissue structures were disorder, and large areas of blue collagen fibers were visible in the infarct area. In the compound of formula (I) administration group, the myocardial tissue structures of the mice were improved to a certain degree, and red myocardial cells and blue collagen were distributed alternately in the infarct area, and collagen fibers were significantly reduced, indicating that the compound of formula (I) reduced the degree of myocardial fibrosis after MI when administered for 28 days, and reduced the degree of fibrosis by 8.8% and 9.6%, respectively, compared with the vehicle control group (panel E). The crystal form A of the compound of formula (I) had substantially the same efficacies.

### Test Example 8: Pharmacodynamic Evaluation of Compound of Formula (I) in Rat Model of Myocardial Ischemia

### 1. Experimental Objective

This study aimed to evaluate the pharmacodynamic effect of the compound of formula (I) in a SD rat model of myocardial ischemia.

### 2. Experimental Methods

After the animals had acclimated to the facility for one week, the model was established by ligation of the left anterior descending coronary artery (LAD). After model establishment, the rats were administered penicillin (80,000 units/rat, i.m.) for 3 days, and disinfected to prevent infection.

One day after model establishment, the modeled animals were randomly divided into 4 groups according to the baseline body weight and echocardiographic imaging indicators, with 4 animals in each sham group (Sham) and 6 animals in each myocardial infarction model group (MI), and drug administration was then initiated. The control group was given the blank vehicle: 5% DMSO + 45% PEG400 + 50% H₂O (1% CMC-Na). The experimental groups were administered by oral gavage at doses of 30 mg/kg and 75 mg/kg, respectively, twice daily for 28 consecutive days. The body weights of the animals were monitored daily after administration, and echocardiographic imaging indicators were measured on days 7 and 28. The primary indicators of echocardiographic evaluation were left ventricular fractional shortening (FS) and left ventricular ejection fraction (LVEF). After the echocardiographic measurement on day 28, heart tissues were collected in a tissue fixative for pathological analysis by Masson's trichrome staining.

### 3. Experimental Results

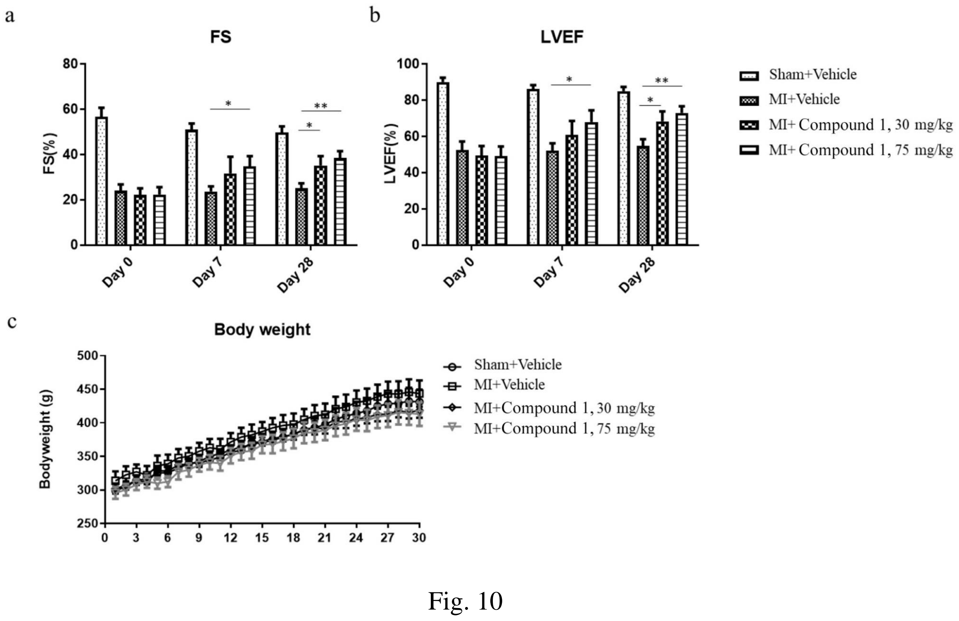

The compound of formula (I) as prepared in Example 1 of the present invention showed good cardiac function-improving activity in the rat model of myocardial infarction (Panels a and b in Figure 10). The compound of formula (I) of the present invention significantly increased the left ventricular ejection fraction (LVEF) and left ventricular fractional shortening (FS) of rats on the 7th day of administration. The 30mpk and 75mpk dosage groups increased the LVEF by 11.43% and 18.85%, respectively, and increased the left ventricular fractional shortening (FS) by 9.73% and 12.62%, respectively. On day 28, the 30mpk and 75mpk dosage groups increased the LVEF by 18.75% and 23.8%, respectively, and increased left ventricular fractional shortening (FS) by 13.01% and 15.88%, respectively. Moreover, a dose-dependent trend was observed, and there was no significant effect on body weight (Panel c). Statistical significance was analyzed using t-test, with p < 0.05 indicating a significant difference.

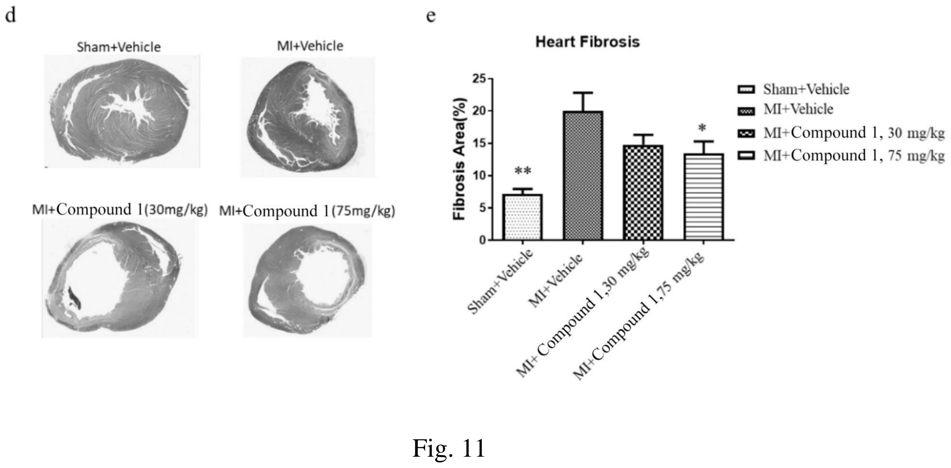

The compound of formula (I) as prepared in Example 1 of the present invention showed an improving effect on myocardial fibrosis in rats with myocardial infarction. Panel d showed an electron micrograph of Masson-stained pathological sections of rat heart tissue. Panel e showed statistical results of the degree of fibrosis in rat heart tissue. As shown in Figure 11, in the Sham group, red myocardial fibers in the rats were arranged neatly, and blue collagen fibers were rarely seen. In the control group of the MI model, myocardial cells were necrotic, myocardial tissue structures were disorder, and large areas of blue collagen fibers were visible in the infarct area. In the compound of formula (I) administration group, the myocardial tissue structures of the rats were improved to a certain degree, and red myocardial cells and blue collagen were distributed alternately in the infarct area, and collagen fibers were significantly reduced, indicating that the compound of formula (I) reduced the degree of myocardial fibrosis after MI when administered for 28 days, and reduced the degree of fibrosis by 5.2% and 6.5%, respectively, compared with the vehicle control group (Panel e). The crystal form A of the compound of formula (I) had substantially the same efficacies.

## Claims

1. A crystal form of a compound of formula (I),

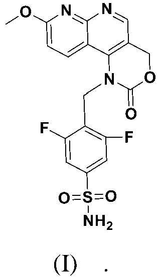

2. The crystal form of the compound of formula (I) according to claim 1, **characterized in that** the crystal form of the compound of formula (I) is a crystal form A, and the crystal form A has an X-ray powder diffraction pattern with Cu-Kα radiation comprising diffraction peaks at the following 2θ angles (±0.2°): 24.9, 25.1, and 25.7;
alternatively, the crystal form A has an X-ray powder diffraction pattern with Cu-Kα radiation comprising diffraction peaks at the following 2θ angles (±0.2°): 12.8, 14.0, 16.0, 18.5, 24.9, 25.1, and 25.7;
alternatively, the crystal form A has an X-ray powder diffraction pattern with Cu-Kα radiation comprising diffraction peaks at the following 2θ angles (±0.2°): 12.8, 14.0, 16.0, 18.5, 24.9, 25.1, 25.7, 26.9, and 28.5;
alternatively, the crystal form A has an X-ray powder diffraction pattern with Cu-Kα radiation comprising diffraction peaks at the following 2θ angles (±0.2°): 12.8, 14.0, 16.0, 18.5, 24.9, 25.1, 25.7, 26.9, 28.5, and 29.8;
alternatively, the crystal form A has an X-ray powder diffraction pattern with Cu-Kα radiation comprising diffraction peaks at the following 2θ angles (±0.2°): 12.8, 14.0, 16.0, 17.0, 18.5, 20.5, 24.9, 25.1, 25.7, 26.9, 28.5, and 29.8;
alternatively, the crystal form A has an X-ray powder diffraction pattern substantially as shown in Figure 1 or Figure 7;
alternatively, the crystal form A has an XRPD pattern analysis with Cu-Kα radiation as shown in Table 1 or Table 2;
alternatively, the crystal form A has a differential scanning calorimetry curve with an endothermic peak at 233.23±4 °C;
alternatively, the crystal form A has a differential scanning calorimetry curve with an exothermic peak at 234.3±4 °C;
alternatively, the crystal form A has a differential scanning calorimetry (DSC) thermogram substantially as shown in Figure 2;
alternatively, the crystal form A has a thermogravimetric analysis curve with a weight loss of 13.50±1%, for example, a weight loss of 13.5111%, in the range of about 105-254 °C;
alternatively, the crystal form A has a thermogravimetric analysis curve with a weight loss of 13.50±1%, for example, a weight loss of 13.5111%, at 254.00±3 °C;
alternatively, the crystal form A of the compound of formula (I) has a thermogravimetric analysis (TGA) thermogram substantially as shown in Figure 3;
alternatively, the crystal form of the compound of formula (I) is a crystal form B, and the crystal form B has an X-ray powder diffraction pattern with Cu-Kα radiation having diffraction peaks at the following 2θ angles (±0.2°): 19.19, 27.18, 13.44, and 22.55;
alternatively, the crystal form B has an X-ray powder diffraction pattern with Cu-Kα radiation having diffraction peaks at the following 2θ angles (±0.2°): 26.19, 20.76, 4.42, 22.84, 19.19, 27.18, 13.44, and 22.55;
alternatively, the crystal form B has an XRPD pattern with Cu-Kα radiation as shown in Figure 4;
alternatively, the crystal form B has an XRPD pattern analysis with Cu-Kα radiation as shown in Table 3;
alternatively, the crystal form B is a DMF solvate of the compound of formula (I);
alternatively, the crystal form of the compound of formula (I) is a crystal form C, and the crystal form C has an X-ray powder diffraction pattern with Cu-Kα radiation having diffraction peaks at the following 2θ angles (±0.2°): 22.11, 22.55, 20.96, and 26.63;
alternatively, the crystal form C has an X-ray powder diffraction pattern with Cu-Kα radiation having diffraction peaks at the following 2θ angles (±0.2°): 22.11, 22.55, 20.96, 26.63, 15.01, 25.68, 26.38, 28.54, 13.22, and 18.68;
alternatively, the crystal form C has an XRPD pattern with Cu-Kα radiation as shown in Figure 5;
alternatively, the crystal form C has an XRPD pattern analysis with Cu-Kα radiation as shown in Table 4;
alternatively, the crystalline form C is a DMSO solvate of the compound of formula (I);
alternatively, the crystal form of the compound of formula (I) is a crystal form D, and the crystal form D has an X-ray powder diffraction pattern with Cu-Kα radiation having diffraction peaks at the following 2θ angles (±0.2°): 22.71, 18.92, 22.96, 26.4, 24.44, 16.62, 13.15, 17.79, and 27.12;
alternatively, the crystal form D has an X-ray powder diffraction pattern with Cu-Kα radiation having diffraction peaks at the following 2θ angles (±0.2°): 22.71, 18.92, 22.96, 26.4, 24.44, 16.62, 13.15, 17.79, 27.12, 24.17, 28.81, 19.95, 28.42, 24.85, 14.78, 17.36, and 29.16;
alternatively, the crystal form D has an XRPD pattern with Cu-Kα radiation as shown in Figure 6;
alternatively, the crystal form D has an XRPD pattern analysis with Cu-Kα radiation as shown in Table 5;
alternatively, the crystal form D is an NMP solvate of the compound of formula (I).

3. A method for preparing the crystal form of the compound of formula (I) according to claim 1 or 2, including method I, comprising:
adding the compound of formula (I) to organic solvent A to dissolve, and then adding the resulting solution to organic solvent B to precipitate crystals;
wherein the organic solvent A is selected from one or more of DMF (*N,N-*dimethylformamide), DMSO (dimethyl sulfoxide), and NMP (N-methylpyrrolidone), and the organic solvent B is selected from one or more of water, methanol, ethanol, MTBE, toluene, and dichloromethane;
in some embodiments of the present invention, when the compound of formula (I) is the crystal form A, in the method I, the organic solvent A is DMF, and the organic solvent B is selected from one or two of water and ethanol; or the organic solvent A is DMSO, and the organic solvent B is selected from one or two of toluene and methanol;
in some embodiments of the present invention, when the compound of formula (I) is the crystal form B, in the method I, the organic solvent A is DMF, and the organic solvent B is selected from one or two of MTBE and toluene;
in some embodiments of the present invention, when the compound of formula (I) is the crystal form D, in the method I, the organic solvent A is NMP, and the organic solvent B is selected from one or more of water, dichloromethane, and MTBE;
or including method II, comprising:
adding the compound of formula (I) to organic solvent C to dissolve, and then adding organic solvent D to precipitate crystals;
wherein the organic solvent C is selected from one or more of DMF, DMSO, and NMP, and the organic solvent D is selected from one or more of acetone, ethylene glycol methyl ether, water, dichloromethane, ethyl acetate, MTBE, toluene, tetrahydrofuran, dioxane, ethanol, trifluoroethanol, acetonitrile, ethylene glycol dimethyl ether, isopropanol, and butanone;
preferably, when the compound of formula (I) is the crystal form A, in the method II, the organic solvent C is DMF, and the organic solvent D is selected from one or more of acetone, ethylene glycol methyl ether, water, and dichloromethane; or the organic solvent C is DMSO, and the organic solvent D is selected from one or more of ethanol, trifluoroethanol, ethylene glycol methyl ether, acetonitrile, dioxane, ethylene glycol dimethyl ether, and butanone; or the organic solvent C is NMP, and the organic solvent D is selected from one or more of water, acetonitrile, dichloromethane, and ethylene glycol dimethyl ether;
preferably, when the compound of formula (I) is the crystal form B, in the method II, the organic solvent C is DMF, and the organic solvent D is selected from one or more of ethyl acetate, MTBE, toluene, tetrahydrofuran, dioxane, and butanone;
preferably, when the compound of formula (I) is the crystal form C, in the method II, the organic solvent C is DMSO, and the organic solvent D is selected from one or more of ethyl acetate, water, and toluene;
preferably, when the compound of formula (I) is the crystal form D, in the method II, the organic solvent C is NMP, and the organic solvent D is selected from one or more of isopropanol, MTBE, toluene, tetrahydrofuran, and butanone;
or including method III, comprising:
suspending the compound of formula (I) in organic solvent E, slurrying and filtering to obtain crystals;
wherein the organic solvent E is selected from one or more of methanol, ethanol, isopropanol, ethyl acetate, n-heptane, MTBE, ethylene glycol methyl ether, water, acetonitrile, toluene, dichloromethane, tetrahydrofuran, dioxane, ethylene glycol dimethyl ether, butanone, dimethylformamide, dimethyl sulfoxide, N-methylpyrrolidone, and acetone;
preferably, when the compound of formula (I) is the crystal form A, in the method III, the organic solvent E is selected from one or more of methanol, ethanol, isopropanol, ethyl acetate, n-heptane, MTBE, ethylene glycol methyl ether, water, acetonitrile, toluene, dichloromethane, tetrahydrofuran, dioxane, ethylene glycol dimethyl ether, and butanone;
preferably, when the compound of formula (I) is the crystal form B, in the method III, the organic solvent E is DMF;
preferably, when the compound of formula (I) is the crystal form C, in the method III, the organic solvent E is DMSO;
preferably, when the compound of formula (I) is the crystal form D, in the method III, the organic solvent E is NMP.

4. A crystalline composition, comprising the crystal form of the compound of formula (I) according to any one of claims 1-3, preferably, the crystal form of the compound of formula (I) in the crystalline composition is selected from one or more of the crystal form A, the crystal form B, the crystal form C, and the crystal form D;
preferably, the crystal form of the compound of formula (I) accounts for 10% or more, 20% or more, 30% or more, 40% or more, 50% or more, 60% or more, 70% or more, 80% or more, 90% or more, 91% or more, 92% or more, 93% or more, 94% or more, 95% or more, 96% or more, 97% or more, 98% or more, 99% or more, or 99.5% or more, by weight of the crystalline composition;
preferably, the crystal form of the compound of formula (I) accounts for 50% or more, 60% or more, 70% or more, 80% or more, 90% or more, 91% or more, 92% or more, 93% or more, 94% or more, 95% or more, 96% or more, 97% or more, 98% or more, 99% or more, or 99.5% or more, by weight of the crystalline composition.

5. A pharmaceutical composition, comprising the crystal form of the compound of formula (I) according to claim 1 or 2 or the crystalline composition according to claim 4, and optionally a pharmaceutically acceptable carrier.

6. The pharmaceutical composition according to claim 5, comprising one, two or more additional drugs for treating cancers or tumours.

7. Use of the crystal form of the compound of formula (I) according to claim 1 or 2, the crystalline composition according to claim 4, or the pharmaceutical composition according to claim 5 or 6 as a medicament or in the preparation of a medicament, wherein the medicament is used to prevent and/or treat ENPP1-mediated diseases.

8. Use of the crystal form of the compound of formula (I) according to claim 1 or 2, the crystalline composition according to claim 4, or the pharmaceutical composition according to claim 5 or 6 as a medicament or in the preparation of a medicament, wherein the medicament is used to prevent and/or treat cancers or tumour-related diseases, or cardiovascular diseases;
preferably, the crystal form of the compound of formula (I) according to claim 1 or 2, the crystalline composition according to claim 4, or the pharmaceutical composition according to claim 5 or 6 is used in combination with one, two or more additional drugs for treating cancers or tumours or a treatment means.

9. The pharmaceutical composition according to claim 6 or the use according to claim 8, wherein the anti-tumour drug is a chemotherapeutic drug, a targeted therapeutic drug or an immunotherapeutic drug;
preferably, the drug for preventing and/or treating cancers or tumours is selected from the group consisting of cell signal transduction inhibitors, chlorambucil, melphalan, cyclophosphamide, ifosfamide, busulfan, carmustine, lomustine, streptozotocin, cisplatin, carboplatin, oxaliplatin, dacarbazine, temozolomide, procarbazine, methotrexate, fluorouracil, cytarabine, gemcitabine, mercaptopurine, fludarabine, vinblastine, vincristine, vinorelbine, paclitaxel, docetaxel, topotecan, irinotecan, etoposide, trabectedin, dactinomycin, doxorubicin, epirubicin, daunomycin, mitoxantrone, bleomycin, mitomycin C, ixabepilone, tamoxifen, flutamide, gonadorelin analogs, megestrol, prednisone, dexamethasone, methylprednisolone, thalidomide, interferon alpha, leucovorin, sirolimus, temsirolimus, everolimus, afatinib, alisertib, amuvatinib, apatinib, axitinib, bortezomib, bosutinib, brivanib, cabozantinib, cediranib, crenolanib, crizotinib, dabrafenib, dacomitinib, danusertib, dasatinib, dovitinib, erlotinib, foretinib, ganetespib, gefitinib, ibrutinib, icotinib, imatinib, iniparib, lapatinib, lenvatinib, linifanib, linsitinib, masitinib, momelotinib, motesanib, neratinib, nilotinib, niraparib, oprozomib, olaparib, pazopanib, pictilisib, ponatinib, quizartinib, regorafenib, rigosertib, rucaparib, ruxolitinib, saracatinib, saridegib, sorafenib, sunitinib, telatinib, tivantinib, tivozanib, tofacitinib, trametinib, vandetanib, veliparib, vemurafenib, erivedge, volasertib, alemtuzumab, bevacizumab, brentuximab vedotin, catumaxomab, cetuximab, denosumab, gemtuzumab, ipilimumab, nimotuzumab, ofatumumab, panitumumab, rituximab, tositumomab, trastuzumab, PI3K inhibitors, CSF1R inhibitors, A2A and/or A2B receptor antagonists, IDO inhibitors, anti-PD-1 antibodies, anti-PD-L1 antibodies, LAG3 antibodies, TIM-3 antibodies, TIGIT antibodies, CD47 antibodies, CLAUDIN 18.2 antibodies, anti-CTLA-4 antibodies or any combination thereof; and preferably, the treatment means is radiotherapy or surgery.

10. The use according to claim 8, **characterized in that** the cancers or tumours are selected from the group consisting of skin cancers, bladder cancers, ovarian cancers, breast cancers, gastric cancers, pancreatic cancers, prostate cancers, colon cancers, lung cancers, bone cancers, brain cancers, neurocytoma, rectal cancers, colon cancers, familial adenomatous polyposis, hereditary non-polyposis colorectal cancers, esophageal cancers, lip cancers, laryngeal cancers, hypopharyngeal cancers, tongue cancers, salivary gland cancers, adenocarcinoma, medullary thyroid carcinomas, papillary thyroid cancers, kidney cancers, renal parenchymal cancers, cervical cancers, corpus uteri cancers, endometrial cancers, choriocarcinoma, testicular cancers, urinary cancers, melanoma, brain tumours, such as glioblastoma, astrocytoma, meningioma, medulloblastoma and peripheral neuroectodermal tumours, Hodgkin's lymphoma, non-Hodgkin's lymphoma, Burkitt's lymphoma, leukemia, acute lymphoblastic leukemia (ALL), chronic lymphoblastic leukemia (CLL), acute myeloid leukemia (AML), chronic myeloid leukemia (CML), adult T-cell leukemia lymphoma, diffuse large B-cell lymphoma (DLBCL), hepatocellular carcinoma, gallbladder cancers, bronchogenic carcinoma, small cell lung cancers, non-small cell lung cancers, multiple myeloma, basal cell tumours, teratoma, retinoblastoma, choroidal melanoma, seminoma, rhabdomyosarcoma, craniopharyngioma, osteosarcoma, chondrosarcoma, myosarcoma, liposarcoma, fibrosarcoma, Ewing's sarcoma and plasmacytoma; and the cardiovascular disease is selected from the group consisting of myocardial infarction (MI), heart failure (HF), cardiac trauma, abnormal scar formation in cardiac tissue, cardiomyopathy, myocardial cell death, enhanced cardiac repair, and the release of pro-inflammatory molecules in myocardial cells.
